# EUROPÄISCHE PATENTSCHRIFT

(11) **EP 1 373 272 B1**
(45) Veröffentlichungstag und Bekanntmachung des Hinweises auf die Patenterteilung: **29.06.2005**
(21) Anmeldenummer: 02735166.7
(22) Anmeldetag: 20.03.2002
(51) Int. Cl.: C07D 487/06, C07D 209/56, C07D 241/38, C07D 487/22, C09B 69/10

(54) **TERT.-ALKYLPHENOXYSUBSTITUIERTE POLYCYCLISCHE VERBINDUNGEN**
TERT. ALKYLPHENOXY SUBSTITUTED POLYCYCLIC COMPOUNDS
COMPOSES POLYCYCLIQUES SUBSTITUES PAR ALKYLPHENOXY TERTIAIRE

(30) Priorität: 23.03.2001 US 278009 P
(43) Veröffentlichungstag der Anmeldung: 02.01.2004
(73) Patentinhaber: BASF AKTIENGESELLSCHAFT, 67056 Ludwigshafen (DE)
(72) Erfinder: BÖHM, Arno, 68305 Mannheim (DE); HELFER, Willi, 67159 Friedelsheim (DE); BECK, Georg, 67482 Böbingen (DE); KRIEGER, Matthias, 79539 Lörrach (DE); ERK, Peter, 67227 Frankenthal (DE)
(86) Internationale Anmeldenummer: PCT/EP2002/003279
(87) Internationale Veröffentlichungsnummer: WO 2002/076988

(56) Entgegenhaltungen:
- EP-A- 0 638 614
- EP-A- 0 675 489
- WO-A-01/42331
- GB-A- 2 290 489
- HOFKENS, J. ET AL: "Conformational rearrangements in and twisting of a single molecule" CHEMICAL PHYSICS LETTERS (2001), 333(3,4), 255-263 , XP002219188
- DATABASE CA [Online] CHEMICAL ABSTRACTS SERVICE, COLUMBUS, OHIO, US; KITAGAWA, SUMIKO ET AL: "Optical recording medium" retrieved from STN Database accession no. 126:150636 XP002219193 & JP 08 300814 A (TDK ELECTRONICS CO LTD, JAPAN) 19. November 1996 (1996-11-19)
- XU, HUIJUN ET AL: "Aspects of metal phthalocyanine photosensitization systems for light energy conversion" JOURNAL OF PHOTOCHEMISTRY AND PHOTOBIOLOGY, A: CHEMISTRY (1992), 65(1-2), 267-76 , XP008010041
- WURTHNER, FRANK ET AL: "Fluorescent and electroactive cyclic assemblies from perylene tetracarboxylic acid bisimide ligands and metal phosphine triflates" CHEMISTRY--A EUROPEAN JOURNAL (2001), 7(4), 894-902 , XP002219189
- C. FORMER ET AL.: "Cyclodehydrogenation of poly(perylene) to poly(quaterrylene): toward poly(pery-naphthalene)" MACROMOLECULES., Bd. 35, Nr. 5, 2002, Seiten 1576-1582, XP002219190 AMERICAN CHEMICAL SOCIETY, EASTON, PA., US ISSN: 0024-9297
- A. HERRMANN ET AL.: "Polyphenylene dendrimers with perylene diimide as a luminescent core" CHEMISTRY - A EUROPEAN JOURNAL., Bd. 7, Nr. 22, 2001, Seiten 4844-4853, XP002219191 VCH PUBLISHERS., US ISSN: 0947-6539
- KOCH M ET AL: "REVERSIBLY CROSSLINKED NETWORKS OF NANOPARTICLES IN METALLOCENE-CATALYZED OLEFIN POLYMERIZATION" MACROMOLECULAR: RAPID COMMUNICATIONS, WILEY VCH, WEINHEIM, DE, Bd. 22, Nr. 17, 11. Dezember 2001 (2001-12-11), Seiten 1455-1462, XP001113456 ISSN: 1022-1336
- WURTHNER, FRANK ET AL: "Fluorescent J-type aggregates and thermotropic columnar mesophases of perylene bisimide dyes" CHEMISTRY--A EUROPEAN JOURNAL (2001), 7(10), 2245-2253 , XP002219192

## Beschreibung

Die vorliegende Erfindung betrifft neue tert.-alkylphenoxysubstituierte polycyclische Verbindungen der allgemeinen Formel I 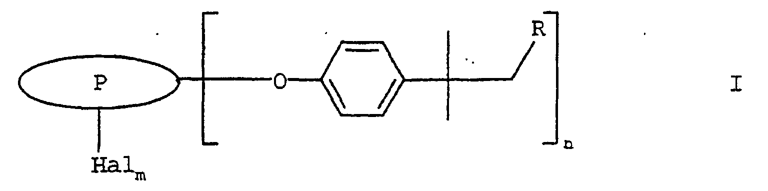 in der die Variablen folgende Bedeutung haben:
- P: ein gegenüber Basen und Nukleophilen stabiler, gegebenenfalls Arylsubstituenten tragender, konjugierter polycyclischer Rest, der keine Gruppierungen aus der Gruppe -CO-NH-CO-, -COOH und -CO-O-CO- enthält;
- R: C₁-C₈-Alkyl, dessen Kohlenstoffkette durch eine oder mehrere Gruppierungen -O-, -S-, -NR¹-, -CO- und/oder -SO₂- unterbrochen sein kann und das durch C₁-C₆-Alkoxy oder einen über ein Stickstoffatom gebundenen, 5- bis 7-gliedrigen heterocyclischen Rest, der weitere Heteroatome enthalten und aromatisch sein kann, ein- oder mehrfach substituiert sein kann; C₅-C₈-Cycloalkyl, dessen Kohlenstoffgerüst durch eine oder mehrere Gruppierungen -O-, -S-, -NR¹-, -CO- und/oder -SO₂- unterbrochen sein kann und das gegebenenfalls ein- oder mehrfach durch C₁-C₆-Alkyl substituiert sein kann;
- R¹: Wasserstoff oder C₁-C₆-Alkyl;
- Hal: Chlor und/oder Brom;
- m: eine Zahl von 0 bis 15;
- n: eine Zahl von 1 bis 16, wobei die Summe m + n ≤ 16 ist,
sowie die Herstellung dieser Verbindungen und ihre Verwendung zur Einfärbung von hochmolekularen organischen und anorganischen Materialien, als Dispergierhilfsmittel und Pigmentadditive für organische Pigmente, als farbgebende Komponente in der dekorativen Kosmetik und zur Herstellung farbiger oder im ultravioletten und/oder nahinfraroten Bereich des elektromagnetischen Spektrums absorbierender wäßriger Polymerisatdispersionen.

Polycyclische organische Verbindungen bereiten aufgrund ihrer geringen Löslichkeiten und geringen Kompatibilitäten oftmals Schwierigkeiten bei der Einarbeitung in Anwendungsmedien. Dieses Problem wirkt sich insbesondere bei Pigmenten, Fluoreszenzfarbstoffen und UV-Absorbern aus, für deren Farbstärke, Fluoreszenz bzw. UV-Schutzwirkung gute Verteilbarkeit im Anwendungsmedium wesentlich ist.

Zur reversiblen Solubilisierung amino- bzw. iminogruppenhaltiger Chromophore wird in den EP-A-648 817, EP-A-648 770 und WO-A-98/32802 die Einführung thermisch wieder abspaltbarer Alkoxycarbonylsubstituenten an den Amino- bzw. Imidstickstoffatomen beschrieben. Diese Methode ist jedoch auf NH-Gruppierungen enthaltende Chromophore beschränkt und ergibt im allgemeinen nur in mäßig polaren Medien und nur für geringe Chromophorkonzentrationen von < 1 Gew.-% eine brauchbare Solubilisierung bzw. Kompatibilisierung. Außerdem verhindert die thermische Fragmentierungsneigung der Carbamatfunktion den Einsatz in hochschmelzenden Thermoplasten wie Polymethylmethacrylat, Polyethylenterephthalat und Polycarbonat. Auch die in der DE-A-37 13 459 beschriebene Derivatisierung von Diketopyrrolopyrrolen mit tertiären Butylgruppen führt zu den gleichen Einschränkungen hinsichtlich der Einsatzmedien und Chromophorkonzentrationen.

Aus den WO-A-96/22331, EP-A-227 980, WO-A-97/22607 und WO-A-96/22332 sind Perylen-3,4-dicarbonsäuremonoimide, Perylen-3,4:9,10-tetracarbonsäurediimide und Quaterrylen-3,4:13,14-tetracarbonsäurediimide bekannt, die im Ringgerüst durch bis zu 4 Kohlenstoffatome enthaltende Alkylreste substituierte Phenoxyreste als Substituenten tragen. Auch diese modifizierten Chromophore weisen nur in Anwendungsmedien mittlerer Polarität ausreichende Löslichkeit auf.

In Adv. Mater. 11, S. 754-758 (1999) wird die Ausbildung mesoskopischer Superstrukturen durch Wechselwirkung von im Perylengerüst aryloxysubstituiertem Perylen-3,4:9,10-tetracarbonsäurediimid und N-(2-Ethylhexyl)perylen-3,4:9,10-tetracarbonsäurediimid mit langkettige Alkylreste tragendem Melamin in organischen Lösungsmitteln wie Chloroform, Tetrachlorkohlenstoff und Methylcyclohexan untersucht. In diesem Artikel wird auf die schlechte Löslichkeit der 1,6,7,12-tetraphenoxy-, -(p-tert.-butyl)phenoxy und -(p-tert.-octyl)phenoxysubstituierten, am Imidstickstoffatom unsubstituierten Perylen-3,4:9,10-tetracarbonsäurediimide hingewiesen. Die beschriebenen Perylenderivate unterscheiden sich von den erfindungsgemäßen Verbindungen dadurch, daß sie aufgrund des unsubstituierten Stickstoffatoms nicht basenstabil sind.

In Chem. Mater., 12, S. 352-362 (2000) wird die über Sol-Gel-Prozesse ablaufende Einbindung von an den Imidstickstoffatomen alkoxysilan-modifizierten (und daher ebenfalls nicht basenstabilen) sowie teilweise zusätzlich im Perylengerüst aryloxysubstituierten Perylen-3,4:9,10-tetracarbonsäurediimiden und Perylen-3,4-dicarbonsäureimiden in anorganische Netzwerke untersucht. Dabei wurde festgestellt, daß die Löslichkeit der Perylenderivate durch die Kombination einer Modifizierung der Imidstickstoffatome mit einer 3-Triethoxysilylpropylgruppe und einer Substitution des Perylengerüsts mit p-1,1,3,3-Tetramethylbutylphenoxygruppen gesteigert werden kann.

Der Erfindung lag die Aufgabe zugrunde, neue polycyclische Effektstoffe mit deutlich verbessertem Löslichkeitsverhalten in unpolaren wie polaren Medien (Breitbandkompatibilität) sowie einer deutlich verringerten Aggregationsneigung bereitzustellen.

Demgemäß wurden die tert.-alkylphenoxysubstituierten polycyclischen Verbindungen der eingangs definierten Formel I gefunden.

Außerdem wurde ein Verfahren zur Herstellung der Verbindungen I gefunden, welches dadurch gekennzeichnet ist, daß man ein Halogenid der allgemeinen Formel II 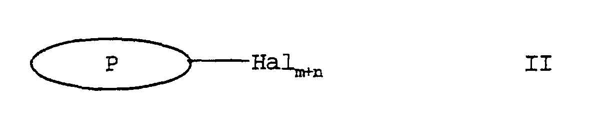 in einem inerten stickstoffbasischen Lösungsmittel in Anwesenheit einer Base mit einem tert.-Alkylphenol der allgemeinen Formel III 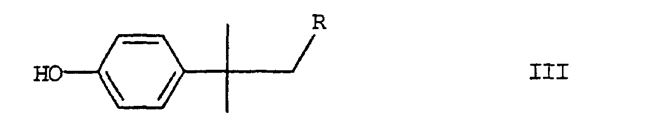 umsetzt und gegebenenfalls ungewünschtes Halogen anschließend entfernt.

Weiterhin wurde die Verwendung der Verbindungen I zur Einfärbung von hochmolekularen organischen und anorganischen Materialien, als Dispergierhilfsmittel und Pigmentadditiv für organische Pigmente, als farbgebende Komponente in der dekorativen Kosmetik sowie zur Herstellung farbiger oder im ultravioletten und/oder nahinfraroten Bereich des elektromagnetischen Spektrums absorbierender wäßriger Polymerisatdispersionen gefunden.

Die Verbindungen der Formel I basieren auf einem bei den Reaktionsbedinungen gegenüber Basen und Nukleophilen stabilen, konjugierten polycyclischen Rest P, der keine Gruppierungen aus der Gruppe -CO-NH-CO-, -COOH und -CO-O-CO- enthält.

Der Rest P kann weitere Arylsubstituenten tragen, die basisch nicht angreifbar sind, z.B. unsubstituiertes, alkyl- und/oder alkoxysubstituiertes Aryl, insbesondere Phenyl, oder Hetaryl, wie 2-, 3- und 4-Pyridyl und Pyrimidyl. Diese Arylsubstituenten können entweder direkt an das Ringgerüst oder bei den weiter unten aufgeführten polycyclischen Imiden auch an die Imidstickstoffatome gebunden sein.

Der bzw. die tert.-Alkylphenoxyreste können bei solchen arylsubstituierten Resten P auch über die Arylsubstituenten an den Rest P gebunden sein, also z.B. bei Diphenyldiketopyrrolopyrrol oder N,N'-Diphenylperylen-3,4:9,10-tetracarbonsäurediimid über die 4-oder 3,5-Positionen des Phenylrestes.

Vorzugsweise bedeutet P einen basenstabilen Rest aus der Gruppe der Naphthaline, Anthracene, Phenanthrene, Tetracene, Perylene, Terrylene, Quaterrylene, Pentarylene und Hexarylene, Anthrachinone, Indanthrone, N-substituierten Naphthalin-1,8-dicarbonsäuremonoimide (im folgenden kurz "Naphthalmonoimide" genannt), N,N'disubstituierten Naphthalin-1,8:4,5-tetracarbonsäurediimide (kurz "Naphthalimide"), N-substituierten Perylen-3,4-dicarbonsäuremonoimide (kurz "Perylmonoimide"), N,N'-disubstituierten Perylen-3,4:9,10-tetracarbonsäurediimide (kurz "Perylimide"), N,N'disubstituierten Terrylen-3,4:11,12-tetracarbonsäurediimide (kurz "Terrylimide"), N,N'-disubstituierten Quaterrylen-3,4:13,14-tetracarbonsäurediimide (kurz "Quaterrylimide"), Acridine, Carbazole, Dibenzofurane, Dinaphthofurane, Benzimidazole, Benzthiazole, Phenazine, Dioxazine, Chinacridone, Metallphthalocyanine, Metallnaphthalocyanine, Metallporphyrine, Cumarine, Dibenzofuranone, Dinaphthofuranone, Benzimidazolone, Indigoverbindungen, Thioindigoverbindungen, Chinophthalone, Naphthochinophthalone und Diketopyrrolopyrrole.

Besonders bevorzugt sind dabei Reste P aus der Gruppe der Naphthaline, Chinacridone, Diketopyrrolopyrrole, Dioxazine, Indanthrone, Metallphthalocyanine, Metallnaphthalocyanine, Naphthalmonoimide, Perylmonoimide, Perylimide, Terrylimide und Quaterrylimide, wobei die Metallphthalocyanine, Metallnaphthalocyanine, Metallporphyrine, Terrylimide und Quaterrylimide ganz besonders bevorzugt sind.

Die die Verbindungen I charakterisierenden tert.-Alkylphenoxyreste sowie die gegebenenfalls zusätzlich vorhandenen Halogenatome können direkt oder, wie oben bereits beschrieben, über gegebenenfalls vorhandene Arylsubstituenten an das Ringgerüst der Reste P gebunden sein. Selbstverständlich können auch beide Bindungsformen in einer Verbindung I auftreten. Größere Reste P, wie Perylmonoimide, Perylimide, Terrylimide und Quaterrylimide, tragen die tert.-Alkylphenoxyreste bevorzugt direkt am Ringgerüst oder weisen zumindest direkt gebundene tert.-Alkylphenoxyreste zusätzlich zu arylengebundenen tert.-Alkylphenoxyresten auf.

Je nach Größe des konjugierten Ringgerüsts enthalten die Verbindungen I mindestens 1 und bis zu 16 (n: 1 bis 16), insbesondere 2 bis 8, tert.-Alkylphenoxyreste.

Beim erfindungsgemäßen Verfahren werden die tert.-Alkylphenoxyreste durch Halogenaustausch in die Verbindungen I eingeführt. Dementsprechend können die Verbindungen I bei nicht vollständigem Austausch der Halogenatome auch bis zu 15 (m: 0 bis 15), vor allem 1 bis 4, Halogenatome enthalten, wobei die Gesamtzahl beider Substituentengruppen 16, vorzugsweise 8, nicht überschreiten sollte.

Beispielhaft seien für die besonders bevorzugten Reste P die generell geeigneten und die bevorzugten Bereiche für die Summe m+n genannt: Naphthaline: 1 bis 4, insbesondere 1 bis 2; Chinacridone: 1 bis 8, insbesondere 2 bis 4; Diketopyrrolopyrrole: 1 bis 6, insbesondere 2 bis 4; Dioxazine: 1 bis 8, insbesondere 2 bis 4; Indanthrone: 1 bis 6, insbesondere 2 bis 4; Metallphthalocyanine: 1 bis 16, insbesondere 4 bis 8; Metallnaphthalocyanine: 1 bis 16, insbesondere 8 bis 16; Naphthalmonoimide: 1 bis 4, insbesondere 1 bis 2; Perylmonoimide: 1 bis 6, insbesondere 1 bis 3; Perylimide: 1 bis 8, insbesondere 2 bis 6; Terrylimide: 1 bis 12, insbesondere 2 bis 8; Quaterrylimide: 1 bis 14, insbesondere 2 bis 8.

Weisen die Reste P zusätzliche Arylsubstituenten auf, die nicht zur Bindung der tert.-Alkylphenoxyreste dienen, erniedrigt sich natürlich die maximal mögliche Summe m+n entsprechend.

Im folgenden sind geeignete Beispiele für die in Formel I auftretenden Reste R und R¹ sowie für deren Substituenten aufgeführt:
Methyl, Ethyl, Propyl, Isopropyl, Butyl, Isobutyl, tert.-Butyl, Pentyl, Isopentyl, Neopentyl, tert.-Pentyl, Hexyl, 2-Methylpentyl, tert.-Pentyl, Hexyl, 2-Methylpentyl, Heptyl, 1-Ethylpentyl, Octyl, 2-Ethylhexyl und Isooctyl;
2-Methoxyethyl, 2-Ethoxyethyl, 2-Propoxyethyl, 2-Isopropoxyethyl, 2-Butoxyethyl, 2- und 3-Methoxypropyl, 2- und 3-Ethoxypropyl, 2-und 3-Propoxypropyl, 2- und 3- Butoxypropyl, 2- und 4-Methoxybutyl, 2- und 4-Ethoxybutyl, 2- und 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- und 4-Butoxybutyl, 4,8-Dioxadecyl, 3,6,9-Trioxadecyl und 3,6,9-Trioxaundecyl;
2-Methylthioethyl, 2-Ethylthioethyl, 2-Propylthioethyl, 2-Isopropylthioethyl, 2-Butylthioethyl, 2- und 3-Methylthiopropyl, 2- und 3-Ethylthiopropyl, 2- und 3-Propylthiopropyl, 2- und 3- Butylthiopropyl, 2- und 4-Methylthiobutyl, 2- und 4-Ethylthiobutyl, 2-und 4-Propylthiobutyl, 3,6-Dithiaheptyl, 3,6-Dithiaoctyl, 4,8-Dithianonyl, 3,7-Dithiaoctyl, 3,7-Dithianonyl, 4,7-Dithiaoctyl, 4,7-Dithianonyl, 2- und 4-Butylthiobutyl, 4,8-Dithiadecyl, 3,6,9-Trithiadecyl und 3,6,9-Trithiaundecyl;
2-Monomethyl- und 2-Monoethylaminoethyl, 2-Dimethylaminoethyl, 2-und 3-Dimethylaminopropyl, 3-Monoisopropylaminopropyl, 2- und 4-Monopropylaminobutyl, 2- und 4-Monomethylaminobutyl, 6-Methyl-3,6-diazaheptyl, 3,6-Dimethyl-3,6-diazaheyptyl, 3,6-Diazaoctyl, 3,6-Dimethyl-3,6-diazaoctyl, 9-Methyl-3,6,9-triazadecyl, 3,6,9-Trimethyl-3,6,9-triazadecyl, 3,6,9-Triazaundecyl und 3,6,9-Trimethyl-3,6,9-triazaundecyl;
Propan-2-on-1-yl, Butan-3-on-1-yl, Butan-3-on-2-yl und 2-Ethyl-pentan-3-on-1-yl;
2-Methylsulfonylethyl, 2-Ethylsulfonylethyl, 2-Propylsulfonylethyl, 2-Isopropylsulfonylethyl, 2-Buthylsulfonylethyl, 2- und 3-Methylsulfonylpropyl, 2- und 3-Ethylsulfonylpropyl, 2- und 3-Propylsulfonylpropyl, 2- und 3-Butylsulfonylpropyl, 2- und 4-Methylsulfonylbutyl, 2- und 4-Ethylsulfonylbutyl, 2- und 4-Propylsulfonylbutyl und 4-Butylsulfonylbutyl;
Methoxy, Ethoxy, Propoxy, Isopropoxy, Butoxy, Isobutoxy, sec.-Butoxy, tert.-Butoxy, Pentoxy, Isopentoxy, Neopentoxy, tert.-Pentoxy und Hexoxy;
Cyclopentyl, 2- und 3-Methylcyclopentyl, 2- und 3-Ethylcyclopentyl, Cyclohexyl, 2-, 3- und 4-Methylcyclohexyl, 2-, 3- und 4-Ethylcyclohexyl, 3- und 4-Propylcyclohexyl, 3- und 4-Isopropylcyclohexyl, 3- und 4-Butylcyclohexyl, 3- und 4-sec.-Butylcyclohexyl, 3- und 4-tert.-Butylcyclohexyl, Cycloheptyl, 2-, 3- und 4-Methylcycloheptyl, 2-, 3- und 4-Ethylcycloheptyl, 3- und 4-Propylcycloheptyl, 3- und 4-Isopropylcycloheptyl, 3- und 4-Butylcycloheptyl, 3- und 4-sec.-Butylcycloheptyl, 3- und 4-tert.-Butylcycloheptyl, Cyclooctyl, 2-, 3-, 4- und 5-Methylcyclooctyl, 2-, 3-, 4- und 5-Ethylcyclooctyl, 3-, 4- und 5-Propylcyclooctyl, 2-Dioxanyl, 4-Morpholinyl, 2- und 3-Tetrahydrofuryl, 1-, 2- und 3-Pyrrolidinyl und 1-, 2-, 3- und 4-Piperidyl.

Als Beispiele für bevorzugte tert.-Alkylphenoxyreste seien p-(1,1-Dimethylpropyl)phenoxy, p-(1,1-Dimethylbutyl)phenoxy, p-(1,1-Dimethylpentyl)phenoxy, p-(1,1,3,3-Tetramethylbutyl)phenoxy, p-(2-Cyclopentyl-1,1-dimethylethyl)phenoxy, p-(2-Cyclohexyl-1,1-dimethylethyl)phenoxy, p-(2-Cycloheptyl-1,1-dimethylethyl)phenoxy und p-(1,1-Dimethyl-2-(4-morpholinyl)ethyl)phenoxy genannt.

Die besonders bevorzugten Naphthalmonoimide, Perylmonoimide, Perylimide, Terrylimide und Quaterrylimide tragen insbesondere folgende basenstabile Substituenten an den Imidstickstoffatomen:
C₆-C₃₀-Alkyl, dessen Kohlenstoffkette durch eine oder mehrere Gruppierungen -O-, -S-, -NR¹-, -CO- und/oder -SO₂- unterbrochen sein kann und das durch C₁-C₆-Alkoxy oder einen über ein Stickstoffatom gebundenen, 5- bis 7-gliedrigen heterocyclischen Rest, der weitere Heteroatome enthalten und aromatisch sein kann, ein-oder mehrfach substituiert sein kann;
C₅-C₈-Cycloalkyl, dessen Kohlenstoffgerüst durch eine oder mehrere Gruppierungen -O-, -S-, -NR¹-, -CO- und/oder -SO₂- unterbrochen sein kann und das gegebenenfalls ein- oder mehrfach durch C₁-C₆-Alkyl substituiert sein kann;
Aryl oder Hetaryl, das jeweils ein- oder mehrfach durch C₁-C₁₈-Alkyl, C₁-C₆-Alkoxy, Cyano, -CONH-R¹ und/oder -NH-COR¹ substituiert sein kann.

Im einzelnen seien für diese Substituenten ergänzend zu den bereits genannten Resten folgende Reste beispielhaft aufgeführt:
Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl und Eicosyl (die Bezeichnungen Isononyl, Isodecyl und Isotridecyl sowie die weiter oben angeführte Bezeichnung Isooctyl sind Trivialbezeichnungen und stammen von den nach der Oxosynthese erhaltenen Alkoholen);
3,6,9-Trioxadodecyl, 3,6,9,12-Tetraoxatridecyl und 3,6,9,12-Tetraoxatetradecyl; 3,6,9-Trithiadodecyl, 3,6,9,12-Tetrathiatridecyl und 3,6,9,12-Tetrathiatetradecyl;
Carbamoyl, Methylaminocarbonyl, Ethylaminocarbonyl, Propylaminocarbonyl, Butylaminocarbonyl, Pentylaminocarbonyl, Hexylaminocarbonyl, Heptylaminocarbonyl, Octylaminocarbonyl, Nonylaminocarbonyl und Decylaminocarbonyl; Formylamino, Acetylamino und Propionylamino;
2-, 3- und 4-Methylphenyl, 2,4-, 2,5-, 3,5- und 2,6-Dimethylphenyl, 2,4,6-Trimethylphenyl, 2-, 3- und 4-Ethylphenyl, 2,4-, 2,5-, 3,5- und 2,6-Diethylphenyl, 2,4,6-Triethylphenyl, 2-, 3- und 4-Propylphenyl, 2,4-, 2,5-, 3,5- und 2,6- Dipropylphenyl, 2,4,6-Tripropylphenyl, 2-, 3- und 4-Isopropylphenyl, 2,4-, 2,5-, 3,5- und 2,6-Diisopropylphenyl, 2,4,6-Triisopropylphenyl, 2-, 3-und 4-Butylphenyl, 2,4-, 2,5-, 3,5- und 2,6-Dibutylphenyl, 2,4,6-Tributylphenyl, 2-, 3- und 4-Isobutylphenyl, 2,4-, 2,5-, 3,5- und 2,6-Diisobutylphenyl, 2,4,6-Triisobutylphenyl, 2-, 3-und 4-sec.-Butylphenyl, 2,4-, 2,5-, 3,5- und 2,6-Di-sec.-butylphenyl, 2,4,6-Tri-sec.-butylphenyl, 2-, 3- und 4-tert.-Butylphenyl, 2,4-, 2,5-, 3,5- und 2,6-Di-tert.-butylphenyl und 2,4,6-Tri-tert.-Butylphenyl; 2-, 3- und 4-Methoxyphenyl, 2,4-, 2,5-, 3,5-und 2,6-Dimethoxyphenyl, 2,4,6-Trimethoxyphenyl, 2-, 3- und 4-Ethoxyphenyl, 2,4-, 2,5-, 3,5- und 2,6-Diethoxyphenyl, 2,4,6-Triethoxyphenyl, 2-, 3- und 4-Propoxyphenyl, 2,4-, 2,5-, 3,5- und 2,6-Dipropoxyphenyl, 2-, 3- und 4-Isopropoxyphenyl, 2,4-, 2,5-, 3,5- und 2,6-Diisopropoxyphenyl und 2-, 3- und 4-Butoxyphenyl; 2-, 3- und 4-Cyanophenyl; 3- und 4-Carboxamidophenyl, 3- und 4-N-(Methyl)carboxamidophenyl und 3- und 4-N-(Ethyl)carboxamidophenyl; 3- und 4-Acetylaminophenyl, 3- und 4-Propionylaminophenyl und 3- und 4-Butyrylaminophenyl.

Die erfindungsgemäßen Verbindungen I können vorteilhaft nach dem ebenfalls erfindungsgemäßen Verfahrenen hergestellt werden, indem man die entsprechenden Halogenide der Formel II mit tert.-Alkylphenolen der Formel III in einem inerten stickstoffbasischen Lösungsmittel in Anwesenheit einer Base umsetzt und gegebenenfalls ungewünschtes Halogen anschließend entfernt.

Als inertes stickstoffbasisches Lösungsmittel eignen sich hierfür insbesondere polare Lösungsmittel, vor allem Stickstoffheterocyclen, wie Pyridin, Pyrimidin, Chinolin, Isochinolin, Chinaldin und vorzugsweise N-Methylpyrrolidon, sowie Carbonsäureamide, wie N,N-Dimethylformamid und N,N-Dimethylacetamid.

Die Lösungsmittelmenge beträgt abhängig von der Löslichkeit des Halogenids II üblicherweise 2 bis 40 g, vorzugsweise 4 bis 25 g, Lösungsmittel je g Halogenid II.

Als Base sind insbesondere nicht oder nur schwach nukleophile Verbindungen geeignet. Beispiele für solche Basen sind Alkalimetallhydroxide, wie Kalium- und Natriumhydroxid, Alkalimetallcarbonate, wie Kalium- und Natriumcarbonat, sowie Alkalimetallalkoholate tertiärer Alkohole, wie Lithium-, Natrium- und Kalium-tert.-butylat, die in wasserfreier Form zum Einsatz kommen.

In der Regel werden 0,8 bis 1,5, vorzugsweise 1,0 bis 1,2, Moläquivalente Base je mol zu substituierendes Halogenatom eingesetzt.

Die als Ausgangsstoffe eingesetzten Halogenide II sind allgemein bekannt oder nach bekannten Methoden durch Umsetzung der nichthalogenierten konjugierten polycyclischen Verbindungen mit Halogenierungsmitteln, insbesondere den elementaren Halogenen, erhältlich. Halogenide II, die die Halogenatome an Arylsubstituenten gebunden enthalten, sind bekanntermaßen in der Regel über die Einführung der halogenierten Arylreste in das polycyclische System zugänglich.

Das Molverhältnis Halogenid II zu Phenol III hängt von der Anzahl der zu substituierenden Halogenatome ab. Im allgemeinen verwendet man 1 bis 2, bevorzugt 1 bis 1,3, mol Phenol III je mol auszutauschendes Halogenatom im Halogenid II.

Die Reaktionstemperatur liegt üblicherweise im Bereich von 50 bis 200°C, vorzugsweise bei 60 bis 140°C.

Es empfiehlt sich, die Umsetzung unter Schutzgas, z.B. Stickstoff oder Argon, durchzuführen.

Die Reaktionszeit beträgt in Abhängigkeit von der Reaktivität des Halogenids II etwa 2 bis 48 h.

Durch Auswahl der Reaktionsbedingungen - Menge an Phenol III und Base sowie der Reaktionstemperatur - kann der Halogenaustausch vorteilhaft gesteuert werden, so daß sowohl Produkte I, in denen alle Halogenatome ausgetauscht sind (m=0), als auch Produkte I, die noch Halogen enthalten, problemlos hergestellt werden können. Gewünschtenfalls kann das Halogen anschließend noch aus dem Produkt I entfernt werden. Aus einem Ausgangsprodukt II können also verschiedene Produkte I nach Wunsch hergestellt werden.

Verfahrenstechnisch geht man zweckmäßigerweise so vor, daß man das Lösungsmittel vorlegt, Halogenid II, Phenol III und Base zugibt und die erhaltene Lösung bzw. Suspension unter Rühren unter Schutzgas 2 bis 48 h auf die gewünschte Reaktionstemperatur erhitzt.

Die Isolierung des Reaktionsprodukts kann nach Abkühlen auf Raumtemperatur durch direktes Abfiltrieren des ausgefallenen Reaktionsprodukts oder durch Abfiltrieren nach Verdünnen mit dem 3-4fachen Volumen an Wasser, einer verdünnten anorganischen Säure, z.B. 5 bis 10 gew.-%iger Salzsäure, oder eines aliphatischen Alkohols, z.B. Methanol, Waschen zunächst mit wenig Lösungsmittel und anschließend mit Wasser bis zum neutralen Ablauf und Trocknen im Vakuum erfolgen.

In manchen Fällen, insbesondere zur Erzielung hoher Substitutionsgrade n bei den basenlabileren und damit stärker zu unerwünschten Nebenreaktionen neigenden Bromiden II, kann es für die Erzielung einer hohen Produktreinheit von Vorteil sein, die Phenoxylierungsreaktion zweistufig durchzuführen. Hierbei setzt man das Halogenid II zunächst nur mit einer Teilmenge, zweckmäßigerweise der zum Austausch der labilsten Halogensubstituenten benötigten Menge, an Phenol III und Base um, trennt das teilphenoxylierte Produkt durch Filtration aus dem Reaktionsgemisch ab und setzt dieses anschließend mit der restlichen Menge Phenol III und Base zum gewünschten Produkt um.

In der Regel haben die erfindungsgemäß erhaltenen Verbindungen I bereits einen so hohen Wertgehalt (> 95%), daß auf eine weitere Reinigung verzichtet werden kann. Analysenreine Produkte können durch Umkristallisation aus aromatischen Lösungsmitteln, wie Toluol und Xylol, oder halogenierten Kohlenwasserstoffen, wie Methylenchlorid und Chloroform, oder durch Filtration einer Lösung der Produkte in diesen Lösungsmitteln über Kieselgel und anschließendes Einengen hergestellt werden.

Wenn nur ein Teil des Halogens ausgetauscht wurde und das noch enthaltene Halogen entfernt werden soll, so kann dies mithilfe bekannter Verfahren geschehen.

Beispielhaft seien für diesen Zweck die beiden folgenden Enthalogenierungsvarianten beschrieben, die besonders vorteilhaft angewendet werden können.

Bei der ersten Variante erfolgt die Enthalogenierung baseninduziert in Gegenwart eines inerten stickstoffbasischen oder aromatischen Lösungsmittels.

Hierfür geeignete Basen sind z.B. Alkalimetallhydroxide, wie Kalium- und Natriumhydroxid, Alkalimetallcarbonate, wie Kalium- und Natriumcarbonat, Alkalimetallalkoholate sekundärer und tertiärer Alkohole wie Lithium-, Natrium- und Kaliumisopropylat und -tert.-butylat, sowie sterisch gehinderte Stickstoffbasen, wie Diazabicyclooctan (DABCO), Diazabicyclononen (DBN) und Diazabicycloundecen (DBU).

Die Menge an Base ist an sich nicht kritisch. Üblicherweise werden 1 bis 3, vorzugsweise 1 bis 1,5, Moläquivalente Base je mol zu eliminierendes Halogenatom eingesetzt.

Als Lösungsmittel können neben aromatischen Lösungsmitteln, wie Toluol und Xylol, die gleichen Lösungsmittel wie bei der Phenoxylierung verwendet werden, deren Einsatzmenge wieder von der Löslichkeit der zu enthalogenierenden Verbindung I abhängt und in der Regel 2 bis 50 g, bevorzugt 5 bis 25 g, je g Verbindung I beträgt.

Die Reaktionstemperatur liegt üblicherweise im Bereich von 50 bis 200°C, vorzugsweise bei 60 bis 130°C.

Es empfiehlt sich, die Enthalogenierung unter Schutzgas, z.B. Stickstoff oder Argon, durchzuführen.

Die Reaktionszeit beträgt in Abhängigkeit von der Reaktivität der zu enthalogenierenden Verbindung I etwa 1 bis 6 h.

Verfahrenstechnisch geht man zweckmäßigerweise so vor, daß man eine Lösung bzw. Suspension der zu enthalogenierenden Verbindung I im Lösungsmittel vorlegt, die Base zugibt und die erhaltene Mischung unter Rühren unter Schutzgas 1 bis 6 h auf die gewünschte Reaktionstemperatur erhitzt. Wenn unerwünschte Nebenreaktionen, z.B. Verseifungen, zu befürchten sind, ist es von Vorteil, die Base erst nach dem Erhitzen auf Reaktionstemperatur zuzugeben.

Die Isolierung des Reaktionsprodukts kann nach Abkühlen auf Raumtemperatur durch Verdünnen des Reaktionsgemischs mit dem dreibis vierfachen Volumen an einer verdünnten anorganischen Säure, z.B. 5 bis 10 gew.-%iger Salzsäure, Abfiltrieren des so ausgefällten Produkts, Waschen zunächst mit der verdünnten Säure und anschließend mit Methanol oder Wasser bis zum neutralen Ablauf und Trocknen im Vakuum erfolgen.

Bei der zweiten Variante wird eine übergangsmetallkatalysierte reduktive Enthalogenierung in Gegenwart eines bei den Reaktionsbedingungen inerten Lösungsmittels vorgenommen.

Als Reduktionsmittel werden hierbei bevorzugt komplexe Hydride, insbesondere Aluminiumhydride, wie Lithiumaluminiumhydrid, und vor allem Borhydride, bevorzugt Natriumborhydrid, oder elementarer Wasserstoff eingesetzt.

Die Reduktionsmittelmenge ist an sich nicht kritisch. Im allgemeinen kommen 1 bis 5, vorzugsweise 2 bis 3, Moläquivalente Reduktionsmittel je mol zu eliminierendes Halogenatom zum Einsatz.

Als Übergangsmetallkatalysator eignen sich insbesondere Palladiumverbindungen, wobei Pd(II)- und Pd(0)-Verbindungen verwendet werden können. Bei der Reduktion mit komplexen Hydriden sind Palladium(II)acetat, Dichloro(1,5-cyclooctadien)palladium(II), Dichloro[1,1'-bis(diphenylphosphino)ferrocen]palladium(II), Tris(dibenzylidenaceton)dipalladium(0), Tetrakis(triphenylphosphin)palladium(0) und Tetrakis(tris-o-tolylphosphin)palladium(0) bevorzugt, für die Reduktion mit elementarem Wasserstoff empfiehlt sich die Verwendung palladiumdotierter Aktivkohle.

Im allgemeinen werden 0,5 bis 2 mol-% Katalysator je mol zu eliminierendes Halogenatom eingesetzt.

Die Art des Lösungsmittels hängt von dem gewählten Reduktionsmittel ab. Bei Verwendung der komplexen Hydride sind insbesondere polare, aprotische Lösungsmittel, wie aliphatische und cycloaliphatische Ether, aromatische Lösungsmittel und aliphatische Nitrile, und bei Einsatz von elementarem Wasserstoff vor allem aliphatische Alkohole geeignet.

Als Beispiele für diese Lösungsmittel seien im einzelnen Diethylether, Tetrahydrofuran und Dioxan, Toluol und Xylol, Acetonitril, das insbesondere zusammen mit Borhydriden zum Einsatz kommt, sowie Methanol und Ethanol genannt.

Die Lösungsmittelmenge wird durch die Löslichkeit der zu enthalogenierenden Verbindung I bestimmt und liegt in der Regel bei 2 bis 50 g, vorzugsweise 5 bis 25 g, je g Verbindung I.

Die Reaktionstemperatur beträgt üblicherweise 0 bis 150°C, vor allem 20 bis 100°C, wobei die Reduktion mit komplexen Hydriden im allgemeinen bei höheren Temperaturen (etwa 50 bis 100°C) durchgeführt wird als die Reduktion mit elementarem Wasserstoff.

Bei Verwendung der komplexen Hydride als Reduktionsmittel empfiehlt es sich, unter Schutzgas zu arbeiten. Bei der Hydrierung mit elementarem Wasserstoff wird zweckmäßigerweise ein geringer Wasserstoffüberdruck verwendet.

In Abhängigkeit von der Reaktivität der Verbindung I nimmt die Enthalogenierung 4 bis 72 h in Anspruch.

Verfahrenstechnisch geht man bei der Enthalogenierung mit komplexen Hydriden zweckmäßigerweise so vor, daß man das Lösungsmittel vorlegt, zu enthalogenierende Verbindung I und Hydrid zugibt und die erhaltene Lösung bzw. Suspension unter Rühren unter Schutzgas 4 bis 72 h auf die gewünschte Reaktionstemperatur erhitzt.

Nach Abkühlen auf Raumtemperatur und Zerstörung von überschüssigem Hydrid durch Zugabe von Wasser kann die Isolierung des Reaktionsprodukts wie bei der baseninduzierten Enthalogenierung beschrieben vorgenommen werden.

Bei der Enthalogenierung mit elementarem Wasserstoff geht man verfahrenstechnisch zweckmäßigerweise so vor, daß man eine Suspension von zu enthalogenierender Verbindung I und Katalysator im Lösungsmittel in einem Hydrierreaktor vorlegt und unter Rühren unter geringem Wasserstoffüberdruck (etwa 0,1 bis 0,5 bar) 4 bis 72 h auf die Reaktionstemperatur erhitzt.

Nach Abkühlen auf Raumtemperatur, Entspannen und Verdrängen von überschüssigem Wasserstoff mit Stickstoff kann man das Reaktionsprodukt wie bereits beschrieben isolieren.

Sind Verbindungen I mit einem Wertgehalt von > 95% erwünscht, können die bei der Enthalogenierung erhaltenen Verbindungen noch einem Reinigungsschritt unterzogen werden. Geeignet sind z.B. eine fraktionierte Kristallisation aus Lösungsmittelgemischen mit einem aromatischen Lösungsmittel, wie Toluol und Xylol, oder einem halogenierten Kohlenwasserstoff, wie Methylenchlorid, Chloroform und 1,1,2,2-Tetrachlorethan, als der einen Komponente und einem extrem unpolaren Lösungsmittel, wie Pentan oder Hexan, als der anderen Komponente oder eine Säulenchromatographie an Kieselgel unter Verwendung dieser Lösungsmittelgemische als Eluens.

Die erfindungsgemäßen Verbindungen I zeichnen sich durch ihre hohe Löslichkeit in bzw. ihre sehr gute Kompatibilität mit sowohl polaren Medien (z.B. aliphatische Alkohole und Ester bzw. Polyacrylaten, Polycarbonaten und Polyestern) als auch unpolaren Medien (z.B. Alkanen bzw. Polyolefinen) aus.

Sie eignen sich hervorragend für eine Vielzahl von Anwendungszwecken, beispielsweise zur Einfärbung oder Additivierung von hochmolekularen organischen und anorganischen Materialien, insbesondere von Kunststoffen, Lacken und Druckfarben und oxidischen Materialien, wie Niedertemperaturkeramik und auf Metalloxiden basierenden Pigmenten bzw. Metalloxide als einzelne Schichten enthaltenden mehrschichtig aufgebauten Interferenzpigmenten, als Dispergierhilfsmittel und Pigmentadditiv für organische Pigmente, als farbgebende Komponente in der dekorativen Kosmetik sowie zur Herstellung farbiger oder im UV und/oder NIR absorbierender wäßriger Polymerisatdispersionen, wobei insbesondere nach dem in der WO-A-99/40123 beschriebenen Verfahren vorgegangen werden kann.

Für Anwendungen, bei denen eine farbige, d.h. im sichtbaren Bereich des elektromagnetischen Spektrums absorbierende, Verbindung I erwünscht ist, eignen sich insbesondere solche Verbindungen I, die einen Rest P aus der Gruppe der Perylene, Terrylene, Quaterrylene, Pentarylene, Hexarylene, Indanthrone, Perylmonoimide, Perylimide, Terrylimide, Dinaphthofurane, Dioxazine, Chinacridone, Metallphthalocyanine, Metallporphyrine, Cumarine, Dinaphthofuranone, Indigoverbindungen, Thioindigoverbindungen, Chinophthalone, Naphthochinophthalone und Diketopyrrolopyrrole enthalten.

Für Anwendungen, die eine farblose oder nur schwach gefärbte, im ultravioletten und/oder nahinfraroten Bereich des elektromagnetischen Spektrums absorbierende Verbindung I erfordern, z.B. die Additivierung von hochmolekularen organischen und anorganischen Materialien, als Dispergierhilfsmittel für organische Pigmente sowie zur Herstellung im UV und/oder im NIR absorbierender wäßriger Polymerisatdispersionen, sind vor allem die Verbindungen I geeignet, die einen Rest P aus der Gruppe der Naphthaline, Anthracene, Phenanthrene, Tetracene, Anthrachinone, Naphthalmonoimide, Naphthalimide, Quaterrylimide, Acridine, Carbazole, Dibenzofurane, Benzimidazole, Benzthiazole, Phenazine, Metallnaphthalocyanine, Dibenzofuranone und Benzimidazolone aufweisen.

Als Pigmentadditiv für organische Pigmente eignen sich neben den farblosen oder nur schwach gefärbten Verbindungen I auch farbige Verbindungen I, deren Eigenfarbe weitgehend mit der Eigenfarbe der zu additivierenden Pigmente übereinstimmt.

### Beispiele

### A) Herstellung von erfindungsgemäßen Verbindungen I

### Beispiele 1 bis 9

Eine Mischung von x g (20 mmol) des Halogenids II, y g des tert.-Alkylphenols III, z g der Base B und a ml N-Methylpyrrolidon wurde unter Rühren in einer Stickstoffatmosphäre t h auf T°C erhitzt.

Nach dem Abkühlen auf Raumtemperatur wurde das ausgefallene Reaktionsprodukt entweder direkt (Beispiel 6) oder nach Verdünnen mit dem dreifachen Volumen an Methanol (Beispiele 1 bis 3), Wasser (Beispiel 8) bzw. 5 gew.-% iger Salzsäure (Beispiele 4, 5, 7 und 9) abfiltriert und mit Wasser bis zum neutralen Ablauf gewaschen. Im Fall der Beispiele 1 bis 3 bzw. 6 wurde das Filtergut vorher noch mit wenig Methanol bzw. wenig N-Methylpyrrolidon gewaschen. Bei den Beispielen 6 und 7 wurde noch eine Säulenfiltration mit Methylenchlorid als Eluens durchgeführt. Die abschließende Trocknung erfolgte in allen Fällen bei 100°C im Vakuum.

Diese Umsetzung führte bei allen Beispielen zum vollständigen Austausch der Halogenatome durch die tert.-Alkylphenoxyreste.

Weitere Einzelheiten zu diesen Versuchen sowie deren Ergebnisse sind in Tabelle 1 zusammengestellt. Die Ausbeute in g bezieht sich dabei auf die Gesamtausbeute, während die Ausbeute in % auf das gewünschte Phenoxylierungsprodukt bezogen ist.

In Tabelle 1 wurden folgende Bezeichungen verwendet:
IIa:N-(2,6-Diisopropylphenyl)-1,6,9-tribromperylen-3,4-dicarbonsäureimid (84%ig; enthält außerdem mono- und dibromiertes N-(2,6-Diisopropylphenyl)perylen-3,4-dicarbonsäureimid
IIb: N,N'-Bis(2,6-diisopropylphenyl)-1,6,7,12-tetrachlorperylen-3,4:9,10-tetracarbonsäurediimid
IIc: N,N'-Bis(2,6-diisopropylphenyl)-1,7-dibromperylen3,4:9,10-tetracarbonsäurediimid
IId:N,N'-Didodecyl-1,7-dibromperylen-3,4:9,10-tetracarbonsäurediimid
IIe:1:1-Gemisch aus N,N'-Bis(2,6-diisopropylphenyl)1,6,8,11,16,18-hexabromquaterrylen-3,4:13,14-tetracarbonsäurediimid und N,N'-Bis(2,6-diisopropylphenyl)1,6,8,11,16,19-hexabromquaterrylen-3,4:13,14-tetracarbonsäurediimid
IIf: chloriertes Indanthron mit einem mittleren Chlorierungsgrad von 2 und einem Gehalt von 30 Gew.-% Tetrachlorindanthron
IIg: chloriertes Kupferphthalocyanin mit einem mittleren Chlorierungsgrad von 3 (Chlorgehalt 15,6 Gew.-%)

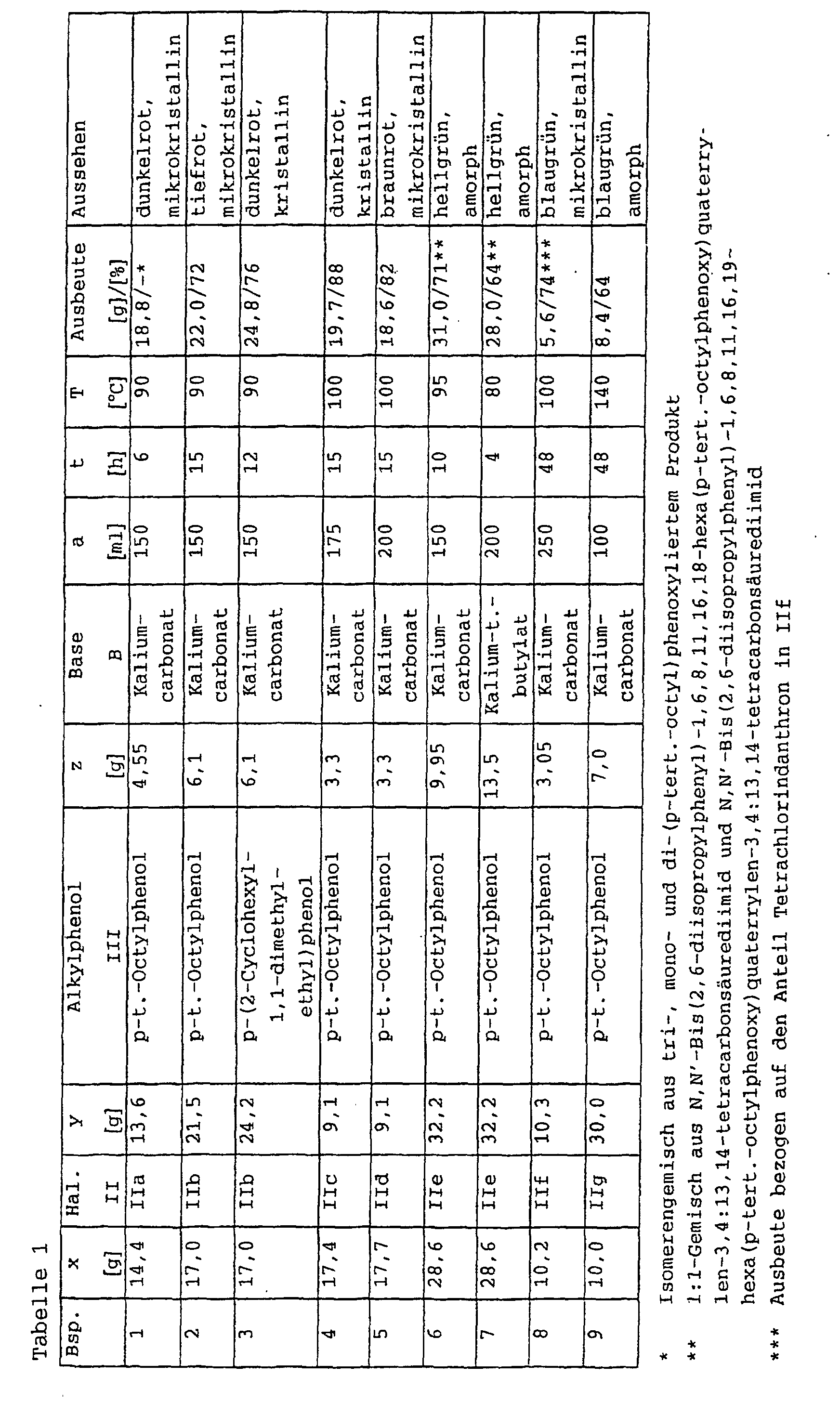

### Beispiel 10

Analog Beipiel 6 wurden 28,6 g eines 1:1-Gemisches von N,N'-Bis(2,6-diisopropylphenyl)-1,6,8,11,16,18-hexabromquaterrylen-3,4:13,14-tetracarbonsäurediimid und N,N'-Bis(2,6-diisopropylphenyl)-1,6,8,11,16,19-hexabromquaterrylen-3,4:13,14-tetracarbonsäurediimid mit 20,6 g p-tert.-Octylphenol und 6,9 g Kaliumcarbonat umgesetzt.

Es wurden 27,6 g eines 1:1-Gemisches von N,N'-Bis(2,6-diisopropylphenyl)-8,18-dibrom-1,6,11,16-tetra(p-tert.-octylphenoxy)quaterrylen-3,4:13,14-tetracarbonsäurediimid und N,N'-Bis(2,6-diisopropylphenyl)-8,19-dibrom-1,6,11,16-tetra(p-tert.-octylphenoxy)-quaterrylen-3,4:13,14-tetracarbonsäurediimid in Form eines dunkelgrünen, kristallinen Pulvers erhalten, was einer Ausbeute von 71% entspricht.

Daneben wurden durch Verdünnen der N-Methylpyrrolidon-Mutterlauge mit der vierfachen Menge eines 1:1-Gemisches von Methanol und Wasser 11 g (Ausbeute 25%) des 1:1-Gemisches aus N,N'-Bis(2,6-diisopropylphenyl)-1,6,8,11,16,18-hexa(p-tert.-octylphenoxy)quaterrylen-3,4:13,14-tetracarbonsäurediimid und N,N'-Bis(2,6-diisopropylphenyl)-1,6,8,11,16,19-hexa(p-tert.-octylphenoxy)quaterrylen-3,4:13,14-tetracarbonsäurediimid aus Beispiel 6 erhalten.

### Beispiel 11

38,7 g des 1:1-Gemisches von N,N'-Bis(2,6-diisopropylphenyl)-8,18-dibrom-1,6,11,16-tetra(p-tert.-octylphenoxy)quaterrylen-3,4:13,14-tetracarbonsäurediimid und N,N'-Bis(2,6-diisopropylphenyl)-8,19-dibrom-1,6,11,16-tetra(p-tert.-octylphenoxy)quaterrylen-3,4:13,14-tetracarbonsäurediimid wurden analog Beispiel 6 mit 10,7 g p-tert.-Octylphenol und 3,32 g Kaliumcarbonat in 170 ml N-Methylpyrrolidon umgesetzt, wobei die Reaktionszeit auf 18 h verlängert wurde.

Es wurden 39,5 g eines 1:1-Gemisches von N,N'-Bis(2,6-diisopropylphenyl)-1,6,8,11,16,18-hexa(p-tert.-octylphenoxy)quaterrylen-3,4:13,14-tetracarbonsäurediimid und N,N'-Bis(2,6-diisopropylphenyl)-1,6 , 8,11, 16, 19-hexa (p-tert.-octylphenoxy)quaterrylen-3,4:13,14-tetracarbonsäurediimid in Form eines hellgrünen, amorphen Pulvers mit einem Restbromgehalt < 0,05 Gew.-% erhalten, was einer Ausbeute von 90% entspricht.

### Beispiel 12

Das 1:1-Gemisch aus N,N'-Bis(2,6-diisopropylphenyl)-8,18-dibrom-1,6,11,16-tetra(p-tert.-octylphenoxy)quaterrylen-3,4:13,14-tetracarbonsäurediimid und N,N'-Bis(2,6-diisopropylphenyl)-8,19-dibrom-1,6,11,16-tetra(p-tert.-octylphenoxy)quaterrylen-3,4:13,14-tetracarbonsäurediimid aus Beispiel 10 (im folgenden als "Ia" bezeichnet) wurde durch Enthalogenierung in N,N'-Bis(2,6-diisopropylphenyl)-1,6,11,16-tetra(p-tert.-octylphenoxy)quaterrylen-3,4:13,14-tetracarbonsäurediimid überführt. Die Enthalogenierung wurde nach den folgenden Methoden vorgenommen:
a) Eine Mischung aus 38,7 g Ia und 600 ml N-Methylpyrrolidon wurde unter Rühren in einer Stickstoffatmosphäre auf 130°C erhitzt, mit 6,75 g Kalium-tert.-butylat versetzt und 1,5 h bei dieser Temperatur gehalten.
   Nach dem Abkühlen auf Raumtemperatur wurde das Reaktionsprodukt durch Eintragen der Reaktionsmischung in 2 1 einer 5 gew.-%igen Salzsäure ausgefällt, abfiltriert, zunächst mit 5 gew.-%iger Salzsäure bis zum farblosen Ablauf und anschließend mit Wasser bis zum neutralen Ablauf gewaschen und bei 100°C im Vakuum getrocknet. Das isolierte Reaktionsprodukt wurde dann einer Säulenchromatographie an Kieselgel mit einem Gemisch aus Toluol und Hexan im Verhältnis 1:1 als Eluens unterzogen.
   Es wurden 20,0 g N,N'-Bis(2,6-diisopropylphenyl)-1,6,11,16-tetra(p-tert.-octylphenoxy)quaterrylen-3,4:13,14-tetracarbonsäurediimid in Form eines hellgrünen, amorphen Pulvers mit einer UV/VIS-spektroskopisch bestimmten Reinheit von > 99% und einem Restbromgehalt < 0,01% erhalten, was einer Ausbeute von 56% entspricht.
b) Es wurde analog Beispiel 12a) vorgegangen, jedoch wurden anstelle von Kalium-tert.-butylat 4,1 g Kaliumcarbonat als Base eingesetzt. Die Aufarbeitung und Reinigung erfolgte ebenfalls analog Beispiel 12a).
   Es wurden 18,6 g N,N'-Bis(2,6-diisopropylphenyl)-1,6,11,16-tetra(p-tert.-octylphenoxy)quaterrylen-3,4:13,14-tetracarbonsäurediimid in Form eines hellgrünen, amorphen Pulvers mit einer Reinheit von > 99,5 % und einem Restbromgehalt von 0,00% erhalten, was einer Ausbeute von 52% entspricht.
c) Eine Mischung aus 38,7 g Ia, 3,8 g Natriumborhydrid, 0,46 g Tetrakis(triphenylphosphin)palladium(0) und 600 ml Dioxan wurde unter Rühren in einer Stickstoffatmospäre 48 h auf 60°C erhitzt.
   Nach dem Abkühlen auf Raumtemperatur wurde überschüssiges Hydrid durch langsame Zugabe von 10 ml Wasser zersetzt. Die weitere Aufarbeitung und Reinigung erfolgte analog Beispiel 12a) .
   Es wurden 21,7 g N,N'-Bis(2,6-diisopropylphenyl)-1,6,11,16-tetra(p-tert.-octylphenoxy)quaterrylen-3,4:13,14-tetracarbonsäurediimid in Form eines hellgrünen, amorphen Pulvers mit einer Reinheit von > 99% und einem Restbromgehalt von 0,00% erhalten, was einer Ausbeute von 61% entspricht.
d) Eine Mischung aus 38,7 g Ia, 3,8 g Natriumborhydrid, 0,23 g Tetrakis(triphenylphosphin)palladium(0) und 1000 ml Acetonitril wurde unter Rühren in einer Stickstoffatmosphäre 25 h auf 69°C erhitzt.
   Nach dem Abkühlen auf Raumtemperaur wurde überschüssiges Hydrid durch langsame Zugabe von 10 ml Wasser zersetzt. Die weitere Aufarbeitung und Reinigung erfolgte analog Beispiel 12a) .
   Es wurden 29,1 g N,N'-Bis(2,6-diisopropylphenyl)-1,6,11,16-tetra(p-tert.-octylphenoxy)quaterrylen-3,4:13,14-tetracarbonsäurediimid in Form eines hellgrünen, amorphen Pulvers mit einer Reinheit von > 99% und einem Restbromgehalt von 0,00% erhalten, was einer Ausbeute von 82% entspricht.

### Beispiel 13

a) Eine Mischung von 20,85 g (25 mmol) N,N'-Bis(2,6-diisopropylphenyl)terrylen-3,4:11,12-tetracarbonsäurediimid, 20 g (125 mmol) Brom und 1250 ml Chloroform wurde unter Rühren und Lichtausschluß 12 h unter Rückfluß erhitzt. Nach Abkühlen der Reaktionslösung auf Raumtemperatur wurde das Lösungsmittel im Vakuum abgezogen, und das Rohprodukt wurde über Kieselgel mit Dichlormethan als Eluens chromatographiert.
   Es wurden 22,5 g N,N'-Bis(2,6-diisopropylphenyl)-1,6,9,14-tetrabromterrylen-3,4:11,12-tetracarbonsäurediimid in Form eines blauen, kristallinen Feststoffs mit einem Schmelzpunkt >300°C erhalten, was einer Ausbeute von 78% entspricht.
   Analytische Daten:
   Elementaranalyse (Gew.-% ber./gef.):
   C: 60,55/60,7; H: 3,7/3,7; N: 2,45/2,45; O: 5,55/5,6; Br: 27,75/27,55;
   Masse (FD, 8kV): m/z = 1145,3 (M⁺, 100%);
   IR (KBr): ν = 1703 (s, C=O), 1660 (s, C=O) cm⁻¹;
   UV/VIS (CHCl₃): λₘₐₓ (ε) = 559 (15850), 605 (46770), 656 (93330) nm.
b) Eine Mischung von 11,5 g (10 mmol) N,N'-Bis(2,6-diisopropylphenyl)-1,6,9,14-tetrabromterrylen-3,4:11,22-tetracarbonsäurediimid, 10,3 g (50 mmol) p-tert.-Octylphenol, 3,45 g (25 mmol) Kaliumcarbonat und 250 ml N-Methylpyrrolidon wurde 8 h unter Rühren in einer Stickstoffatmosphäre auf 80°C erhitzt. Nach Abkühlen auf Raumtemperatur und Verdünnen des Reaktionsgemischs mit dem dreifachen Volumen einer 5 gew.-%igen Salzsäure wurde das ausgefallene Reaktionsprodukt abfiltriert, mit Wasser neutral gewaschen, getrocknet und einer Säulenfiltration an Kieselgel mit Methylenchlorid als Eluens unterworfen.
   Es wurden 13,2 g N,N'-Bis(2,6-diisopropylphenyl)-1,6,9,14-tetra(p-tert.-octylphenoxy)terrylen-3,4:11,12-tetracarbonsäurediimid in Form eines dunkelblauen, kristallinen Feststoffs mit einem Schmelzpunkt > 300°C erhalten, was einer Ausbeute von 80% entspricht.
   Analytische Daten:
   Elementaranalyse (Gew.-% ber./gef.):
   C: 82,85/82,8; H: 7,7/7,7; N: 1,7/1,7; O: 7,75/7,8;
   Masse (FD, 8kV) : m/z = 1651,2 (M⁺, 100%);
   IR (KBr): ν = 1708 (s, C=O), 1668 (s, C=O) cm⁻¹;
   UV/VIS (CHCl₃): λₘₐₓ (ε) = 628 (52930), 669 (128770) nm.

### Beispiel 14

a) Es wurde analog Beispiel 13a) vorgegangen, jedoch wurde eine Mischung von 18,9 g (25 mmol) N-Cyclohexyl-N'-(2,6-diisopropylphenyl)terrylen-3,4:11,12-tetracarbonsäurediimid, 20 g (125 mmol) Brom und 1250 ml Chloroform eingesetzt.
   Es wurden 19,8 g N-Cyclohexyl-N'-(2,6-diisopropylphenyl)-1,6,9,14-tetrabromterrylen-3,4:11,12-tetracarbonsäurediimid in Form eines blauen, mikrokristallinen Feststoffs mit einem Schmelzpunkt > 300°C erhalten, was einer Ausbeute von 74% entspricht.
   Analytische Daten:
   Elementaranalyse (Gew.-% ber./gef.):
   C: 58,25/58,35; H: 3,4/3,4; N: 2,6/2,6; O: 5,95/6,0;
   Br: 29,8/29,65;
   Masse (FD, 8kV): m/z = 1073,0 (M⁺, 100%);
   IR (KBr): ν = 1705 (s, C=O), 1662 (s, C=O) cm⁻¹;
   UV/VIS (CHCl₃): λₘₐₓ (ε) = 556 (16790), 600 (48290), 652 (90070) nm.
b) Es wurde analog Beispiel 13b) vorgegangen, jedoch wurde eine Mischung von 10,7 g (10 mmol) N-Cyclohexyl-N'-(2,6-diisopropylphenyl)-1,6,9,14-tetrabromterrylen-3,4:11,12-tetracarbonsäurediimid, 10,3 g (50 mmol) p-tert.-Octylphenol, 3,45 g (25 mmol) Kaliumcarbonat und 250 ml N-Methylpyrrolidon eingesetzt, und das Rohprodukt wurde einer Säulenfiltration mit Dichlormethan unterzogen.
   Es wurden 12,9 g N-Cyclohexyl-N'-(2,6-diisopropylphenyl)-1,6,9,14-tetra(p-tert.-octylphenoxy)terrylen-3,4:11,12-tetracarbonsäurediimid in Form eines dunkelblauen, kristallinen Feststoffs mit einem Schmelzpunkt >300°C erhalten, was einer Ausbeute von 82% entspricht.
   Analytische Daten:
   Elementaranalyse (Gew.-% ber./gef.):
   C: 85,9/85,8; H: 8,2/8,3; N: 1,8/1,8; O: 4,1/4,1;
   Masse (FD, 8kV): m/z = 1565,3 (M⁺, 100%);
   IR (KBr): ν = 1709 (s, C=O), 1667 (s, C=O) cm⁻¹;
   UV/VIS (CHCl₃): λₘₐₓ (ε) = 624 (54010), 667 (129770) nm.
B) Untersuchung und Anwendung von erfindungsgemäßen Verbindungen I

### Beispiel 15

Die Löslichkeit der in Beispiel 1 bis 14 hergestellten Verbindungen I wurde in extrem unpolaren bis polaren Lösungsmitteln untersucht. Generell wurde eine gegenüber den nicht tert.-alkylphenoxysubstituierten Verbindungen deutlich gesteigerte Löslichkeit beobachtet. Einzelheiten zu diesen Versuchen sind in Tabelle 2 aufgeführt.

Auch in geschmolzenem Polystyrol, Polymethylmethacrylat und Polycarbonat betrug die Löslichkeit dieser Verbindungen I mindestens 10 Gew.-%.

**Tabelle 2**

| Verbindung I aus Bsp. | Löslichkeit [g/l] in | | |
|---|---|---|---|
| | Pentan (25°C) | Toluol (25°C) | Isopropanol (50°C) |
| 1 | 53 | >250 | 56 |
| 2 | 81 | >400 | 92 |
| 3 | 65 | >350 | 87 |
| 4 | 55 | >250 | 59 |
| 5 | 24 | 155 | 47 |
| 6 | 55 | >500 | 49 |
| 7 | 55 | >500 | 49 |
| 8 | 12 | 88 | 10 |
| 12a | 51 | unbegrenzt | 48 |
| 12b | 51 | unbegrenzt | 48 |
| 12c | 51 | unbegrenzt | 48 |
| 13b | 75 | unbegrenzt | 56 |
| 14b | 72 | unbegrenzt | 52 |

### Beispiel 16

Gemäß Beispiel 25 der WO-A-99/40123 wurden wäßrige Polymerisatdispersionen hergestellt, die 15 Gew.-% des Fluoreszenzfarbmittels aus Beispiel 2 oder 10 bzw. 25 Gew.-% des Nahinfrarotabsorbers aus Beispiel 6 in homogener Verteilung enthielten. Die entsprechenden phenoxysubstituierten Derivate konnten dagegen nur bis zu einer Konzentration von 7 bzw. 1 Gew.-% homogen eingearbeitet werden.

## Patentansprüche

1. Tert.-alkylphenoxysubstituierte polycyclische Verbindungen der allgemeinen Formel I 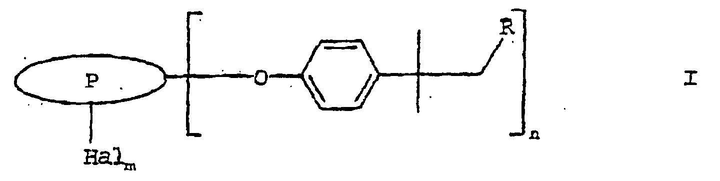 in der die Variablen folgende Bedeutung haben:
P ein gegenüber Basen und Nukleophilen stabiler, gegebenenfalls Arylsubstituenten tragender, konjugierter polycyclischer Rest, der keine Gruppierungen aus der Gruppe -CO-NH-CO-, -COOH und -CO-O-CO- enthält, wobei Metallphthalocyanine und für den Fall, daß n 4 bedeutet, N,N'-disubstituierte Perylen-3,4:9,10-tetracarbonsäurediimide ausgenommen sind;
R C₁-C₈-Alkyl, dessen Kohlenstoffkette durch eine oder mehrere Gruppierungen -O-, -S-, -NR¹-, -CO- und/oder -SO₂- unterbrochen sein kann und das durch C₁-C₆-Alkoxy oder einen über ein Stickstoffatom gebundenen, 5- bis 7-gliedrigen heterocyclischen Rest, der weitere Heteroatome enthalten und aromatisch sein kann, ein- oder mehrfach substituiert sein kann;
C₅-C₈-Cycloalkyl, dessen Kohlenstoffgerüst durch eine oder mehrere Gruppierungen -O-, -S-, -NR¹-, -CO- und/oder -SO₂- unterbrochen sein kann und das gegebenenfalls ein- oder mehrfach durch C₁-C₆-Alkyl substituiert sein kann:
R¹ Wasserstoff oder C₁-C₆-Alkyl;
Hal Chlor und/oder Brom;
m eine Zahl von 0 bis 15 ;
n eine Zahl von 1 bis 16, wobei die Summe m + n ≤ 16 ist.

2. Verbindungen der Formel I nach Anspruch 1, in der P einen basenstabilen Rest aus der Gruppe der Naphthaline, Anthracene. Phenanthrene, Tetracene, Perylene, Terrylene, Quaterrylene, Pentarylene, Hexaxylene, Anthrachinone, Indanthrone, N-substituierten Naphthalin-1,8-dicarbonsäuremonoimide, N,N'-disubstituierten Naphthalin-1,8:4,5-tetracarbonsäurediimide, N-substituierten Perylen-3,4-dicarbonsäuremonoimide, N,N'-disubstituierten Perylen-3,4:9,10-tetracarbonsäurediimide, N,N'-disubstituierten Terrylen-3,4:11,12-tetracarbonsäurediimide, N,N'-disubstituierten Quaterrylen-3,4:13,14-tetracarbonsäurediimide, Acridine, Carbazole, Dibenzofurane, Dinaphthofurane, Benzimidazole, Benzthiazole, Phenazine, Dioxazine, Chinacridone, Metallnaphthalocyanine, Metallporphyrine, Cumarine, Dibenzofuranone, Dinaphthofuranone, Benzimidazolone, Indigoverbindungen, Thioindigoverbindungen, Chinophthalone, Naphthochinophthalone und Diketopyrrolopyrrole bedeutet.

3. Verfahren zur Herstellung von Verbindungen der allgemeinen Formel I gemäß Anspruch 1 oder 2, **dadurch gekennzeichnet, daß** man ein Halogenid der allgemeinen Formel II 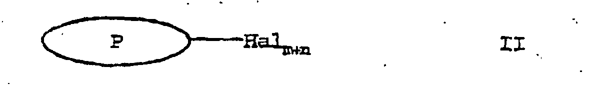 in einem inerten stickstoffbasischen Lösungsmittel in Anwesenheit einer Base mit einem tert.-Alkylphenol der allgemeinen Formel III 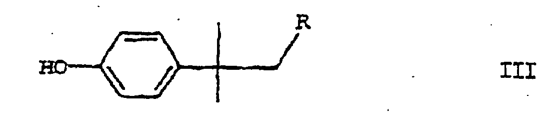 umsetzt und gegebenenfalls ungewünschtes Halogen anschließend entfernt.

4. Verwendung von Verbindungen der allgemeinen Formel I gemäß Anspruch 1 oder 2 zur Einfärbung von hochmolekularen organischen und anorganischen Materialien.

5. Verwendung nach Anspruch 4, **dadurch gekennzeichnet, daß** Kunststoffe, Lacke und Druckfarben eingefärbt werden.

6. Verwendung von Verbindungen der allgemeinen Formel I gemäß Anspruch 1 oder 2 als Dispergierhilfsmittel und Pigmentadditive für organische Pigmente.

7. Verwendung von Verbindungen der allgemeinen Formel I gemäß Anspruch 1 oder 2 als farbgebende Komponente in der dekorativen Kosmetik.

8. Verwendung von Verbindungen der allgemeinen Formel I gemäß Anspruch 1 oder 2 zur Herstellung farbiger oder im ultravioletten und/oder nahinfraroten Bereich des elektromagnetischen Spektrums absorbierender wäßriger Polymerisatdispersionen.

## Claims

1. tert-Alkylphenoxy-substituted polycyclic compounds of the general formula I 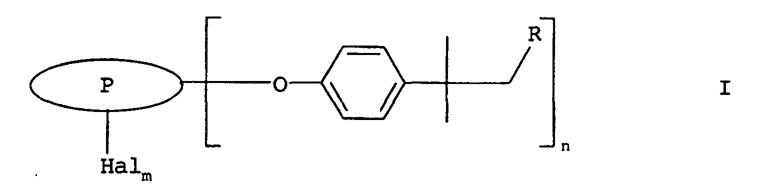 where
P is a conjugated polycyclic radical which is stable to bases and nucleophiles, optionally bears aryl substituents and contains no group from the group consisting of -CO-NH-CO-, -COOH and -CO-O-CO-, although metal phthalocyanines and, if n is 4, N,N'-disubstituted perylene-3,4:9,10-tetracarboxylic diimides shall be excluded;
R is C₁-C₈-alkyl, whose carbon chain may be interrupted by one or more groups selected from the group consisting of -O-, -S-, -NR¹-, -CO- and/or -SO₂- and which may be monosubstituted or polysubstituted by C₁-C₆-alkoxy or by a 5- to 7-membered heterocyclic radical which is attached via a nitrogen atom and may contain further heteroatoms and be aromatic; C₅-C₈-cycloalkyl whose carbon chain may be interrupted by one or more groups selected from the group consisting of -O-, -S-, -NR¹-, -CO- and/or -SO₂- and which may be monosubstituted or polysubstituted by C₁-C₆-alkyl;
R¹ is hydrogen or C₁-C₆-alkyl;
Hal is chlorine and/or bromine;
m is from 0 to 15;
n is from 1 to 16, subject to the proviso that the sum m + n is ≤ 16.

2. Compounds as claimed in claim 1 of the formula I where P is a base-stable radical selected from the group consisting of naphthalenes, anthracenes, phenanthrenes, tetracenes, perylenes, terrylenes, quaterrylenes, pentarylenes, hexarylenes, anthraquinones, indanthrones, N-substituted naphthalene-1,8-dicarboxylic monoimides, N,N'-disubstituted naphthalene-1,8: 4,5-tetracarboxylic diimides, N-substituted perylene-3,4-dicarboxylic monoimides, N,N'-disubstituted perylene-3,4:9,10-tetracarboxylic diimides, N,N'-disubstituted terrylene-3,4:11,12-tetracarboxylic diimides, N,N'-disubstituted quaterrylene-3,4:13,14-tetracarboxylic diimides, acridines, carbazoles, dibenzofurans, dinaphthofurans, benzimidazoles, benzothiazoles, phenazines, dioxazines, quinacridones, metal naphthalocyanines, metal porphyrins, coumarins, dibenzofuranones, dinaphthofuranones, benzimidazolones, indigo compounds, thioindigo compounds, quinophthalones, naphthoquinophthalones and diketopyrrolopyrroles.

3. A process for preparing compounds of the general formula I as set forth in claim 1 or 2, which comprises reacting a halide of the general formula II 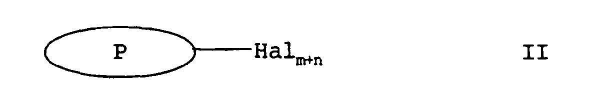 in an inert basic nitrogen-containing solvent in the presence of a base with a tert-alkylphenol of the general formula III 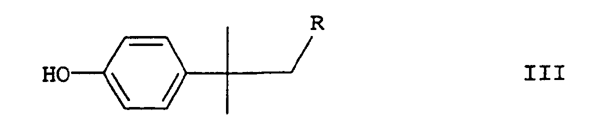 and if desired subsequently removing unwanted halogen.

4. The use of compounds of the general formula I as set forth in claim 1 or 2 for coloring high molecular weight organic and inorganic materials.

5. The use of claim 4, wherein plastics, paints and printing inks are colored.

6. The use of compounds of the general formula I as set forth in claim 1 or 2 as dispersing aids and pigment additives for organic pigments.

7. The use of compounds of the general formula I as set forth in claim 1 or 2 as coloring component in decorative cosmetics.

8. The use of compounds of the general formula I as set forth in claim 1 or 2 for preparing aqueous polymer dispersions that are colored or absorb in the ultraviolet and/or near infrared region of the electromagnetic spectrum.

## Revendications

1. Composés polycycliques substitués par de l'alkylphénoxy tertiaire, répondant à la formule générale I : 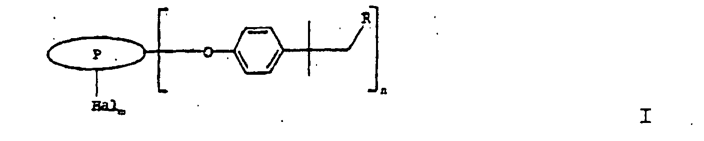 dans laquelle les variables ont la signification suivante :
P représente un radical polycyclique conjugué, stable vis-à-vis des bases et des nucléophiles, qui porte éventuellement des substituants aryle et qui ne contient pas de groupement du groupe -CO-NH-CO-, -COOH et -CO-O-CO-, les phtalocyanines métalliques et, dans le cas où n représente 4, les diimides d'acide pérylène-3,4:9,10-tétracarboxylique N,N'-disubstitués étant exclus,
R représente un groupe alkyle en C₁-C₈, dont la chaîne carbonée peut être interrompue par un ou plusieurs groupements -O-, -S-, -NR¹-, -CO- et/ou -SO₂- et qui peut être substitué à une ou à plusieurs reprises par de l'alcoxy en C₁-C₆ ou par un radical hétérocyclique pentagonal à heptagonal, fixé par un atome d'azote, qui peut contenir d'autres hétéroatomes et être aromatique,
un groupe cycloalkyle en C₅-C₈, dont le squelette carboné peut être interrompu par un ou plusieurs groupements -O-, -S-, -NR¹-, -CO- et/ou -SO₂- et qui peut être substitué éventuellement à une ou à plusieurs reprises par de l'alkyle en C₁-C₆,
R¹ représente de l'hydrogène ou un groupe alkyle en C₁-C₆ ,
Hal représente du chlore et/ou du brome,
m est un nombre de 0 à 15,
n est un nombre de 1 à 16, la somme de m + n ≤ 16.

2. Composés de la formule I suivant la revendication 1, dans laquelle P représente un radical stable vis-à-vis des bases qui est choisi parmi le groupe des naphtalènes, des anthracènes, des phénanthrènes, des tétracènes, des pérylènes, des terrylènes, des quaterrylènes, des pentarylènes, des hexarylènes, des anthraquinones, des indanthrones, des monoimides d'acide naphtalène-1,8-dicarboxylique N-substitués, des diimides d'acide naphtalène-1,8:4,5-tétracarboxylique N,N'-disubstitués, des monoimides d'acide pérylène-3,4-dicarboxylique N-substitués, des diimides d'acide pérylène-3,4:9,10-tétracarboxylique N,N'-disubstitués, des diimides d'acide terrylène-3,4:11,12-tétracarboxylique N,N'-disubstitués, des diimides d'acide quaterrylène-3,4:13,14-tétracarboxylique N,N'-disubstitués, des acridines, des carbazoles, des dibenzofurannes, des dinaphtofurannes, des benzimidazoles, des benzothiazoles, des phénazines, des dioxazines, des quinacridones, des naphtalocyanines métalliques, des porphyrines métalliques, des coumarines, des dibenzofurannones, des dinaphtofurannones, des benzimidazolones, des composés indigo, des composés thioindigo, des quinophtalones, des naphtoquinophtalones et des dicétopyrrolopyrroles.

3. Procédé de préparation de composés de la formule générale I suivant l'une des revendications 1 et 2, **caractérisé en ce que**, dans un solvant basique azoté inerte, on fait réagir, en présence d'une base, un halogénure de la formule générale II : 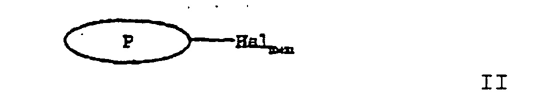 avec un alkylphénol tertiaire de la formule générale III : 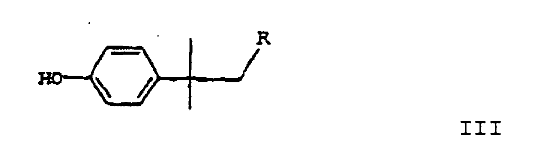 et éventuellement on élimine ensuite de l'halogène non souhaité.

4. Utilisation de composés de la formule générale I suivant l'une des revendications 1 et 2, pour colorer des matières inorganiques et organiques de haut poids moléculaire.

5. Utilisation suivant la revendication 4, **caractérisée en ce qu'**on colore des substances synthétiques, des vernis et des encres d'impression.

6. Utilisation de composés de la formule générale I suivant l'une des revendications 1 et 2, comme adjuvants de dispersion et additifs pour des pigments organiques.

7. Utilisation de composés de la formule générale I suivant l'une des revendications 1 et 2, comme composants colorants dans la cosmétique décorative.

8. Utilisation de composés de la formule générale I suivant l'une des revendications 1 et 2, pour la fabrication de dispersions aqueuses de polymère colorées ou absorbantes dans le domaine ultraviolet et/ou infrarouge proche du spectre électromagnétique.
